# EUROPEAN PATENT SPECIFICATION

(11) **EP 0 481 663 B1**
(45) Date of publication and mention of the grant of the patent: **17.01.1996**
(21) Application number: 91309250.8
(22) Date of filing: 09.10.1991
(51) Int. Cl.: A61L 2/18

(54) **Combined two stage method for cleaning and decontaminating surgical instruments**
Kombiniertes zweistufiges Verfahren zum Reinigen und Entseuchen von chirurgischen Instrumenten
Méthode combinée à deux étapes pour nettoyer et décontaminer des instruments chirurgicaux

(30) Priority: 15.10.1990 US 597651
(43) Date of publication of application: 22.04.1992
(73) Proprietor: E.R. Squibb & Sons, Inc., Princeton, N.J. 08540-4000 (US)
(72) Inventor: Benson, Leslee M., Collinsville, Illinois 62234 (US)
(74) Representative: Mays, Julie

(56) References cited:
- EP-A- 0 268 227
- EP-A- 0 308 786
- EP-A- 0 375 149
- DE-A- 3 327 466
- GB-A- 2 140 819
- US-A- 3 966 408
- US-A- 4 021 377
- WORLD PATENTS INDEX LATEST, Section Ch, Week 9041, Derwent Publications Ltd., London, GB; Class D, AN 90-30903; & JP-A-02 218 605

## Description

### BACKGROUND OF THE INVENTION

### 1. Field of the Invention

The present invention is in the field of cleaning and decontaminating surgical instruments and other medical paraphernalia used in healthcare facilities. This cleaning and decontamination takes place as a preliminary step subsequent to use and soiling of the surgical instruments, and prior to their sterilization by autoclave.

Heretofore, it has been a common practice to merely place used surgical instruments and paraphernalia on towels or in a covered pan prior to their being sent to a central servies facility for sterilization by autoclave, an apparatus which uses superheated steam under pressure to achieve total microbiological decontamination. Thus, no precautions have usually been taken to prevent the transmission of tissue and blood-borne diseases which may take place when personnel working with the soiled surgical instruments accidentally suffer cuts or punctures from these sharp instruments and thereby come in direct contact with blood and tissue on the soiled instruments. Because sterilization was being achieved in the autoclaving process, prior cleaning may have been carried out, but a disinfecting step was either carried out by a separate step or considered redundant, and therefore not used.

More recently, however, there has been widespread concern about the transmission of very serious and often fatal diseases, such as nosocomial infections from resistant strains of bacteria which commonly arise in healthcare facilities, and by viruses carried in tissues and blood, such as hepatitis B and HIV, which may be transmitted to personnel dealing with soiled surgical instruments, as described above.

But, it has been difficult to achieve microbiological decontamination by a method that is vigorous enough to kill resistant strains of Staphylococcus aureus and Pseudomonas aeruginosa, and viruses such as hepatitis B and HIV, while at the same time being simple and straightforward enough to be accomplished with a minimum expenditure of time after the surgical instruments have been used, by personnel requiring a minimum amount of training, and using apparatus and materials which are both inexpensive and reliable. Moreover, it has typically been a problem that the surgical instruments and other paraphernalia may be grossly soiled, and therefore require vigorous cleaning in addition to initial decontamination. Unfortunately, compositions are not available which will accomplish both the vigorous cleaning of grossly soiled surgical instruments and their microbiological decontamination. This problem has been further augmented by the fact that cleaning and decontaminating compositions are usually incompatible for a variety of reasons. Thus, heretofore, it has not been possible to readily combine both the cleaning and decontaminating of surgical instruments into an efficient method.

### 2. Brief Description of the Prior Art

A number of different methods are known in the art for disinfecting or decontaminating surgical instruments and other medical paraphernalia. See, e.g., Bertil Nystrom, 1981 Journal of Hospital Infection, 2, 363-368.

Mild cleaning and disinfecting products are known in the art, e.g., germicidal detergents such as a phenolic-based disinfectant. However, it has not been known or suggested that such germicidal detergents be combined with more vigorous cleaning agents such as enzyme-based presoaks for medical apparatuses and instruments. In the literature for such an enzyme-based presoak, it is usually recommended that any items requiring sterilization or disinfection should be treated in a separate subsequent step. Compatibility of such enzyme-based presoaks is also a recognized problem. For example, it is well known that anionic detergents deactivate protease enzymes.

### SUMMARY OF THE INVENTION

The present invention relates to a method of cleaning and microbiologically decontaminating grossly soiled surgical instruments, comprising the following two steps carried out in the same container: (1) immersing said instruments in an enzyme-based cleaning composition for at least 5 minutes at room temperature, whereby substantially all materials soiling said instruments are removed; followed immediately by (2) adding to said container an amount of a germicidal detergent microbiological decontaminating composition sufficient to give a final concentration of at least 7.8 ml/litre (1 oz./gal.) and continuing to immerse said instruments for at least an additional 10 minutes, whereby substantially all of the microbiological contamination is removed from said instruments.

The present invention further relates to the method of cleaning and microbiologically decontaminating grossly soiled surgical instruments described above in which the cleaning composition and the germicidal detergent microbiological decontaminating composition comprise the following materials:

### ENZYME-BASED CLEANING COMPOSITION

| Ingredient | Percent by Weight |
|---|---|
| Softened Water | 47.0 |
| Borax | 3.3 |
| Triethanol Amine | 12.5 |
| Propylene Glycol | 28.0 |
| Calcium Chloride | 0.1 |
| Citric Acid Anhydrous | 4.0 |
| Linear Alcohol Ethoxylate | 1.0 |
| Protease | 4.0 |
| Hydroxyethyl cellulose | 0.2 |

### GERMICIDAL DETERGENT MICROBIOLOGICAL DECONTAMINATING COMPOSITION

| Ingredient | Percent by Weight |
|---|---|
| Caustic Potash (45% KOH) | 11.4 |
| Sodium Xylene Sulfonate | 4.0 |
| Caustic Soda (50% NaOH) | 3.0 |
| Para Tert-amyl Phenol | 7.8 |
| Phosphoric Acid (75%) | 3.7 |
| Ortho-phenylphenol | 9.2 |
| α-Olefin Sulfonate | 13.4 |
| Fragrance | 0.3 |
| Softened Water | 47.2 |

### DETAILED DESCRIPTION OF THE INVENTION

The term "surgical instruments" as used herein means any of those instuments commonly used in a wide variety of surgical procedures, whether in a hospital operating room environment or in a doctor's office on an outpatient basis. Such instruments are, for the most part, made of surgical quality stainless steel, but they may be composed of other materials as well, e.g., aluminum and polypropylene and other polymer materials. In addition, the term "surgical instruments" includes other medical and surgical paraphernalia which might not properly be considered a surgical instrument, but which comes into contact with human tissue, especially blood, during a surgical or some other medical procedure, during the course of which that item of medical or surgical paraphernalia becomes grossly soiled and microbiologically contaminated. Examples of such medical and surgical paraphernalia are cardiovascular instruments, eye instruments, micro-surgical instruments, neurologic and orthopedic instruments, laparoscopes, flexible fiberoptic scopes, endoscopes, brochoscopes, cystoscopes, and respiratory therapy equipment.

The term "grossly soiled" as used herein means the condition of being contaminated to a substantial extent by contact with human tissue, fluids, excretia, and so forth, as the result of contact therewith during some surgical or other medical procedure. Contamination by contact with human blood in substantial amounts is particularly referred to, and this includes microbiological contamination by viruses and bacteria contained in that blood. A surgical instrument which is "grossly soiled" is one which requires a step of cleaning in addition to a step of decontamination, and is thus one which will benefit in particular from the method of the present invention. The step of cleaning removes the human tissue, fluids, excretia, etc. which have adhered to the surgical instrument, but provides little or no microbiological decontamination of the viruses, bacteria or other microorganisms present in that human tissue, fluids, excretia, etc., which have also adhered to the surgical instrument to be cleaned. For these, the further step of microbiological decontamination in accordance with the method of the present invention is required.

The term "enzyme-based cleaning composition" as used herein refers to cleaning compositions especially designed to remove substantially all human tissue, fluids, excretia, etc. from grossly soiled metal and other surfaces, especially surgical instruments and other medical paraphernalia, in which the enzyme is selected from protease, lipase, amylase, or other enzymes or combinations of enzymes and surfactants known to break down blood, body tissue and excretia.

The enzyme-based cleaning composition may have a number of additional ingredients which help promote its effectiveness and use, e.g., other cleaning agents such as sodium tetraborate, emulsifiers such as triethanolamine, solvent thickeners such as propylene glycol, acidifiers such as citric acid, buffering agents, preservatives, and so forth. Such excipients would be well known to one of ordinary skill in this art.

In a preferred embodiment of the present invention, the enzyme-based cleaning composition has the following composition: protease, non-ionic detergent, propylene glycol, and sodium tetraborate and water.

The term "germicial detergent microbiological decontamination compostion" as used herein refers to germicidal detergents especially designed to provide microbiological decontamination of all grossly soiled metal and other surfaces, especially surgical instruments and other medical paraphernalia, selected from phenolic compounds, quaternary amines, glutaraldehyde and other known disinfectants or combinations thereof.

The disinfectant composition may have a number of additional ingredients which help promote its effectiveness and use, e.g., strong acids and bases such as phosphoric acid and caustic soda, emulsifiers and surfactants such as α-olefin sulfonate, and various fragrances which help to mask the odor of the phenolic compounds.

In a preferred embodiment of the present invention, the germicidal detergent has the following composition: phenol (carbolic acid), anionic detergent, phosphate builders and water.

It is a requirement, but in fact an advantage, of the method of the present invention, that the two steps thereof are carried out in the same container. Once the surgical instruments have been immersed in the bath of enzyme-based cleaning composition, which should be accomplished in such a way that the surgical instruments are completely immersed in said composition, the second step of microbiological decontamination is carried out simply by adding the germicidal detergent to that same container in sufficient amount that the concentration thereof is at least 7.8 ml/litre (1 oz./gal.), with reference to the total volume now present in the container.

The length of time that each step takes has already been indicated as being at least 5 minutes. These minimum immersion times afford the minimum amount of acceptable cleaning and microbiological decontamination. However, longer times are usually desirable, and it is preferred that immersion times of about 20 minutes be utilized for each of the two steps. While longer times may be employed, they do not result in any significant improvement in the extent of cleaning and microbiological decontamination.

The method of the present invention is carried out at room temperature, which is a considerable advantage, since it requires no special provision for heating of the cleaning and/or microbiological decontamination compositions. Room temperature is usually regarded as 20° C, but the method of the present invention is carried out at ambient temperature, whatever that may happen to be. But, of course, temperatures significantly below room temperature will result in a reduced rate of cleaning and microbiological decontamination, requiring correspondingly longer immersion times for each step.

The method of the present invention may be carried out simply by immersing the grossly soiled surgical instruments in the container having the enzyme-based cleaning composition, to which is then added the germicidal detergent microbiological decontamination composition, without any accompanying agitation. However, it will be appreciated that the cleaning and decontamination steps can be accomplished more quickly and efficiently by employing agitation. Consequently, it is also part of the method of the present invention to carry out the steps therof using agitation. Such agitation can be merely manual, or it may be accomplished by use of a mechanical agitating device by means of which the container in which the surgical instruments have been immersed is agitated.

### EXAMPLE OF PREFERRED EMBODIMENT

The following example provides a detailed demonstration of a preferred embodiment of the method of the present invention, but is not intended to be in any way a limitation of the scope therof.
Materials for Gross Soiling: In order to prepare a contamination slurry, a ratio of 5 g of USDA grade A ground sirloin to 20 ml of defibrinated sheeps' blood was placed in a Waring Blender, and blended at high speed for about 2 min, after which the resulting slurry was removed and filtered twice through a sterile filter comprising two layers of 2x2 in². pieces of sterile gauze into sterile 25 x 150 mm test tubes. There was added an equal volume of a 48 hour culture (approximately 10⁹ organisms/ml.) of a microorganism selected from the following:
Staphylococcus aureus ATCC 6538;
Pseudomonas aeruginosa ATCC 15442;
Salmonella choleraesuis ATCC 10708
Propagation of cultures of the above microorganisms and the use of subculture media and other related equipment were as specified in sections 4.001, 4.002, 4.007, 4.008 and 4.025 (Methods 1, 2, and 3) of the AOAC Manual of Methods (14th ed.).
Soiling of Surgical Instruments: To serve as "surgical instruments" there was used polished stainless steel cylinders (penicillin cups) 8 ± 1 mm od, 6 ± 1 mm id, length 10 ± 1 mm of type 304 stainless steel SS 18-8. In order to produce gross soiling of the cylinders, there was used 3 ml of the contamination slurry to which was added 1 ml of the slurry for each cylinder in the test. Thus, for three cylinders, 6 ml of contamination slurry was used. Prior to contamination, the test cylinders were soaked overnight in 1N NaOH, after which they were rinsed with tap water until the rinse water was neutral to phenolphthalein, and then rinsed twice with distilled water. The cleaned cylinders were placed in groups of 10 to 30 and covered with distilled water, sterilized 20 min at 121° C, cooled, and held at room termperature.

To accomplish gross soiling, the required number of cylinders for the test were transferred into the appropriate amount of contamination slurry, and allowed to soak therein at room temperature for 15 min, with occasional gentle shaking to insure that all cylinders were in contact with the slurry. After the 15 min contact period, the liquid was decanted and the cylinders were removed and placed on end in vertical position in a sterile glass petri dish matted with filter paper, covered, and dried in an incubator at 35 ± 1° C for 30 min. The cylinders were transferred using a suitable wire sterilized by flame.
Cleaning Step: The dried cylinders were removed from the petri dish and placed in a sterilized test tube, five cylinders per test tube, to which was then added 10 ml of a enzyme-based cleaning composition containing the following ingredients:

| Ingredient | Percent by Weight |
|---|---|
| Softened Water | 47.0 |
| Borax | 3.3 |
| Triethanol Amine | 12.5 |
| Propylene Glycol | 28.0 |
| Calcium Chloride | 0.1 |
| Citric Acid Anhydrous | 4.0 |
| Linear Alcohol Ethoxylate | 1.0 |
| Protease | 4.0 |
| Hydroxyethyl cellulose | 0.2 |

The cleaning compostion was diluted to a use concentration of 15.6 ml/litre (2 oz/gal) using 400 ppm hard water (prepared according to 4.027 Method 3 of AOAC Manual of Methods, 14th ed., to the desired hardness of 400 ppm), and was maintained at 25° C for 20 min.
Microbiological Decontamination Step: At the end of the 20 min cleaning contact time, there was added to the test tube containing the cylinders and 10 ml of diluted cleaning composition, 0.16 ml of concentrated germicidal detergent microbiological decontamination composition containing the following ingredients:

| Ingredient | Percent by Weight |
|---|---|
| Caustic Potash (45% KOH) | 11.4 |
| Sodium Xylene Sulfonate | 4.0 |
| Caustic Soda (50% NaOH) | 3.0 |
| Para Tert-amyl Phenol | 7.8 |
| Phosphoric Acid (75%) | 3.7 |
| Ortho-phenylphenol | 9.2 |
| α-Olefin Sulfonate | 13.4 |
| Fragrance | 0.3 |
| Softened Water | 47.2 |

which resulted in an overall concentration of 15.6 ml/litre (2 oz/gal.). A contact time of 20 min was allowed for this decontamination step, after which the test tube was shaken.
Evaluation: After shaking the test tube, 2.5 ml of liquid was removed, 1 ml of which was placed in a test tube containing 9 ml of FTLT neutralizing media (fluid thioglycollate with lecithin and polysorbate emulsifier (Tween®), per 4.001 (d)(5) of AOAC Manual of Methods, 14th ed.). The mixture was stirred with a vortex, and 2.5 ml was removed, a first 1 ml of which was placed in a petri dish and labeled 10¹; and a second 1 ml sample of which was added to another test tube containing 9 ml of FTLT, vortexed and a 1 ml sample removed to make the 10² dilution. This procedure was repeated to give 10¹ - 10⁵ samples.

Agar plates containing 15-20 ml of plate count agar were then poured, allowed to cool, inverted, and incubated at 30° C for 48 hrs. The plate count agar was obtained from Difco Laboratories, Detroit, Michigan, 23.5 g of which was rehydrated in 1 L of distilled water, mixed thoroughly and autoclaved for 15-20 min at 121° C to achieve sterilization as per label instructions. The number of colony forming units (CFU's) on the agar plates was counted either manually or using appropriate automated equipment.

The test tubes containing the test cylinders were shaken and all of the cylinders removed at the time the last sample was taken, as described above. Each cylinder was placed in a test tube containing approximately 10 ml of the FTLT neutralizing media, after which the tube was shaken and incubated at 35 ± 1° C for 48 hrs. Each tube was then recorded either as positive, indicating growth, or negative, indicating no growth.

Tests parallel to those described above were run on the first step, second step, and hardwater blank contaminated cylinders as controls.

The phenol resistance of all of the above cultures was determined in accordance with AOAC 4.001 - 4.006. The results of the active formulations combinations showed no growth in the plate count procedure and no growth in the FTLT subculture media after the 20 min contact time with the second step microbiological decontamination. A total of 10 replicates were done for the second step procedure for each of two different batches of cleaning and disinfectant compositions used, for each microorganism. The results of these tests are summarized in the table of data below.

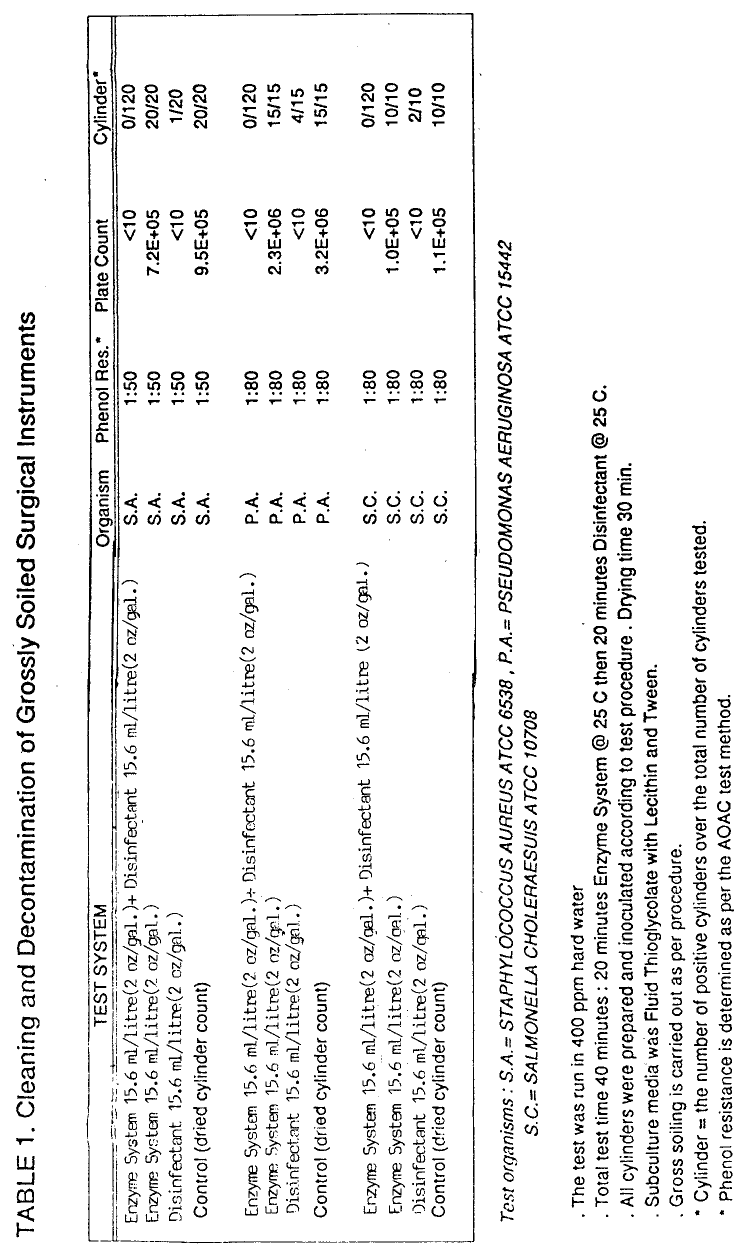

## Claims

1. A method of cleaning and microbiologically decontaminating grossly soiled surgical instruments, comprising the following two steps carried out in the same container: (1) immersing said instruments in an enzyme-based cleaning composition for at least 5 minutes at room temperature, whereby substantially all materials soiling said instruments are removed; followed immediately by (2) adding to said container an amount of a germicidal detergent microbiological decontaminating composition sufficient to give a final concentration of at least 7.8 ml/litre (1 oz./gal.) and continuing to immerse said instruments for at least an additional 5 minutes, whereby substantially all of the soil amd microbiological contamination is removed from said instruments.

2. The method of cleaning and microbiologically decontaminating grossly soiled surgical instruments of Claim 1 in which the enzyme-based cleaning composition and the germicidal detergent microbiological decontaminating composition comprise the following:
| Ingredient | Percent by Weight |
|---|---|
| Softened Water | 47.0 |
| Borax | 3.3 |
| Triethanol Amine | 12.5 |
| Propylene Glycol | 28.0 |
| Calcium Chloride | 0.1 |
| Citric Acid Anhydrous | 4.0 |
| Linear Alcohol Ethoxylate | 1.0 |
| Protease | 4.0 |
| Hydroxyethyl cellulose | 0.2 |
| Ingredient | Percent by Weight |
|---|---|
| Caustic Potash(45% KOH) | 11.4 |
| Sodium Xylene Sulfonate | 4.0 |
| Caustic Soda (50% NaOH) | 3.0 |
| Para Tert-amyl Phenol | 7.8 |
| Phosphoric Acid (75%) | 3.7 |
| Ortho-phenylphenol | 9.2 |
| α-Olefin Sulfonate | 13.4 |
| Fragrance | 0.3 |
| Softened Water | 47.2 |

3. The method of cleaning and microbiologically decontaminating grossly soiled surgical instruments of Claim 1 in which the immersion times for each step is 20 minutes.

4. The method of cleaning and microbioligically decontaminating grossly soild surgical instruments of Claim 2 wherein the concentration of both the cleaning and decontamination compositions is 15.6 ml/litre (2 oz/gal.)

## Patentansprüche

1. Verfahren zur Reinigung und mikrobiologischen Dekontamination von stark verunreinigten chirurgischen Instrumenten, umfassend die folgenden zwei, im selben Behälter ausgeführten Schritte:
(1) Eintauchen der Instrumente in eine Reinigungszusammensetzung auf Enzym-Basis für mindestens 5 Minuten bei Raumtemperatur, wobei im wesentlichen alle die Instrumente verunreinigenden Materialien entfernt werden,
(2) unmittelbar danach Zugabe einer Menge einer keimtötenden, mikrobiologisch dekontaminierenden Detergenszusammensetzung zum Behälter in so ausreichendem Maße, daß eine Endkonzentration von mindestens 7,8 ml/Liter ( 1 oz./gal.) erhalten wird, und Eingetauchtlassen der Instrumente für mindestens weitere 5 Minuten, wobei im wesentlichen die gesamte verunreinigende und mikrobiologische Kontamination von den Instrumenten entfernt wird.

2. Verfahren zur Reinigung und mikrobiologischen Dekontamination stark verunreinigter chirurgischer Instrumente nach Anspruch 1, wobei die Reinigungszusammensetzung auf Enzymbasis und die keimtötende, mikrobiologisch dekontaminierende Detergenszusammensetzung folgende Bestandteile umfassen:
| Bestandteil | Gew.-% |
|---|---|
| enthärtetes Wasser | 47,0 |
| Borax | 3,3 |
| Triethanolamin | 12,5 |
| Propylenglykol | 28,0 |
| Calciumchlorid | 0,1 |
| wasserfreie Citronensäure | 4,0 |
| Lineares Alkoholethoxylat | 1,0 |
| Protease | 4,0 |
| Hydroxyethylcellulose | 0,2 |
| Bestandteil | Gew.-% |
|---|---|
| Ätzkali (45 % KOH) | 11,4 |
| Natriumxylolsulfonat | 4,0 |
| Ätznatron (50 % NaOH) | 3,0 |
| Para-tert.-Amylphenol | 7,8 |
| Phosphorsäure (75 %) | 3,7 |
| Orthophenylphenol | 9,2 |
| α-Olefinsulfonat | 13,4 |
| Aromastoff | 0,3 |
| enthärtetes Wasser | 47,2 |

3. Verfahren zur Reinigung und mikrobiologischen Dekontamination stark verunreinigter chirurgischer Instrumente nach Anspruch 1, wobei die Eintauchzeit bei jedem Schritt 20 Minuten beträgt.

4. Verfahren zur Reinigung und mikrobiologischen Dekontamination stark verunreinigter chirurgischer Instrumente nach Anspruch 2, wobei sowohl die Konzentration der Reinigungs- als auch die der Dekontaminationszusammensetzungen 15,6 ml/Liter (2 oz/gal.) beträgt.

## Revendications

1. Méthode de nettoyage et de décontamination microbiologique d'instruments chirurgicaux grossièrement souillés oomprenant les deux étapes suivantes, effectuées dans le même conteneur : (1) immerger lesdits instruments dans une composition de nettoyage à base d enzymes pendant au moins 5 minutes à température ambiante pour qu'ainsi sensiblement toutes les matières souillant lesdits instruments soient retirées ; et ensuite immédiatement (2) ajouter audit conteneur une quantité d'une composition de décontamination microbiologique détergente germicide suffisante pour donner une concentration finale d'au moins 7,8 ml/litre (1 once/gallon) et continuer à immerger lesdits instruments pendant au moins 5 minutes supplémentaires pour qu'ainsi sensiblement toute la saleté et la contamination microbienne soit retirée desdits instruments.

2. Méthode de nettoyage et de décontamination microbiologique d'instruments chirurgicaux grossièrement souillés selon la revendication 1, où la composition de nettoyage à base d'enzymes et la composition décontaminante microbiologique détergente germicide se composent de ce qui suit :
| Ingrédient | Pour cent en Poids |
|---|---|
| Eau Adoucie | 47,0 |
| Borax | 3,3 |
| Triéthanol Amine | 12,5 |
| Propylène Glycol | 28,0 |
| Chlorure de Calcium | 0,1 |
| Acide Citrique Anhydre | 4,0 |
| Ethoxylate d'Alcool Linéaire | 1,0 |
| Protéase | 4,0 |
| Hydroxyéthyl cellulose | 0,2 |
| Ingrédient | Pour Cent en poids |
|---|---|
| Potasse Caustique (45% KOH) | 11,4 |
| Xylène Sulfonate de Sodium | 4,0 |
| Soude Caustique (50% NaOH) | 3,0 |
| Para Tert-Amyl Phénol | 7,8 |
| Acide Phosphorique (75%) | 3,7 |
| Ortho-phénylphénol | 9,2 |
| α-Oléfine Sulfonate | 13,4 |
| Parfum | 0,3 |
| Eau Adoucie | 47,2 |

3. Méthode de nettoyage et de décontamination microbiologique d'instruments chirurgicaux grossièrement souillés selon la revendication 1, où les temps d'immersion pour chaque étape sont de 20 minutes.

4. Méthode de nettoyage et de décontamination microbiologique d'instruments chirurgicaux grossièrement souillés selon la revendication 2, où la concentration des compositions de nettoyage et de contamination est de 15,6 ml/litre (2 onces/gallon).
